# EUROPEAN PATENT SPECIFICATION

(11) **EP 4 405 005 B1**
(45) Date of publication and mention of the grant of the patent: **02.12.2026**
(21) Application number: 22782725.0
(22) Date of filing: 22.09.2022
(51) Int. Cl.: A61M 5/315, G01D 5/347, A61M 5/24

(54) **DRIVING AN OPTICAL SENSOR OF A DRUG DELIVERY DEVICE OR OF A DRUG DELIVERY ADD-ON DEVICE**
ANSTEUERUNG EINES OPTISCHEN SENSORS EINER ARZNEIMITTELABGABEVORRICHTUNG ODER EINER ARZNEIMITTELABGABEZUSATZVORRICHTUNG
COMMANDE D'UN CAPTEUR TACTILE D'UN DISPOSITIF D'ADMINISTRATION DE MÉDICAMENTS OU D'UN DISPOSITIF COMPLÉMENTAIRE D'ADMINISTRATION DE MÉDICAMENTS

(30) Priority: 24.09.2021 EP 21315176
(43) Date of publication of application: 31.07.2024
(73) Proprietor: Sanofi, 75017 Paris (FR)
(72) Inventor: BROOKES, Glenn, Kendal LA9 5EZ (GB); DRAKE, Jonathan, Loughborough LE11 4SU (GB)
(74) Representative: McDougall, James
(86) International application number: PCT/EP2022/076286
(87) International publication number: WO 2023/046791

(56) References cited:
- WO-A1-2020/094699
- US-A1- 2017 232 203
- US-A1- 2020 360 614
- US-B2- 7 508 436

## Description

### Field

The present disclosure relates to driving an optical sensor of a drug delivery device or of a drug delivery add-on device.

### Background

WO2016131713A1 relates to a data collection device for attachment to an injection device and collecting medicament dosage information therefrom. The data collection device may comprise a mating arrangement configured for attachment to the injection device, a sensor arrangement configured to detect movement of a movable dosage programming component of the injection device relative to the data collection device during delivery of a medicament, and a processor arrangement configured to, based on said detected movement, determine a medicament dosage administered by the injection device. The sensor arrangement may include an optical sensor, for example, an optical encoder unit, particularly including a light source, such as a light emitting diode (LED) and a light detector, such as an optical transducer. The processor arrangement may be configured to monitor a time period elapsed since a pulse was output by the optical encoder and to determine said medicament dosage if said time period exceeds a predetermined threshold.

WO2019101962A1 describes an injection device, which comprises a movable dosage programming component comprising a rotary encoder system having a predefined angular periodicity, and a sensor arrangement comprising a first optical sensor configured to detect movement of the movable dosage programming component relative to the sensor arrangement during dosing of a medicament. The first optical sensor is configured to operate in a strobe-sampling mode at a first frequency. The injection device further comprises a second optical sensor, which is configured to detect movement of the rotary encoder system relative to the second optical sensor and to operate in a strobe-sampling mode at a second frequency lower than the first frequency. Yet further, the injection device comprises a processor arrangement configured to, based on the detected movement of the rotary encoder system, determine a medicament dosage administered by the injection device. The rotary encoder system may be configured to be rotatable with respect to the first optical sensor during a dialing mode of operation of the injection device. The second optical sensor may be configured to operate in a strobe-sampling mode at a second frequency lower than the first frequency. WO2019101962A1 discloses different embodiments of determining a medicament or drug dosage with an optical sensor and a rotary encoder system. Further relevant prior art can be found under US 2020/0360614 A1, which discloses a medicament injection device with optical strobe-sampling sensors and a rotary encoder, US 7,508,436 B2, which discloses CCD/CMOS image sensors with electrical charge clearing before image capture, WO 2020/094699 A1, which discloses a drug delivery assembly with variable sensor sampling frequencies, and US 2017/0232203 A1, which discloses a pen-type drug injector or add-on module with magnetic dosage sensing and high-speed error detection.

### Summary

This disclosure describes methods and devices for driving an optical sensor of a drug delivery device or of a drug delivery add-on device. The invention is defined in the appended claims.

In one aspect the present disclosure provides a method for driving an optical sensor of a drug delivery device or of a drug delivery add-on device, wherein the drug delivery device comprises a movable dosage programming component and the optical sensor is provided and configured to detect movement of the movable dosage programming component relative to the sensor arrangement during dosing of a drug by generating measurement light pulses and detecting reflections of the measurement light pulses from the movable dosage programming component, and wherein the method comprises configuring the optical sensor to generate the measurement light pulses with at least one predefined pulse rate and to generate at least one priming light pulse before one or more measurement light pulses, wherein the at least one priming light pulse is generated with one of the at least one predefined pulse rate, wherein particularly the at least one priming light pulse is only used to bring the optical sensor in a state suited for making measurements with the one or more measurement light pulses, particularly a kind of "steady state". When the priming light pulse is generated with the selected predefined pulse rate, a signal behaviour of the optical sensor generating the measurement light pulses with the selected predefined pulse rate is stimulated. With the at least one priming light pulse, the optical sensor may be in principle brought into a kind of "steady state" and, thus, be rendered more independent from a selected one of the at least one predefined pulse rate. The priming light pulse differs from a measurement light pulse in particular in that it is not used for making measurements, but for "preparing" the optical sensor for measurements, particularly by bringing it into the kind of "steady state". For example, when the optical sensor comprises a light pulse emitter and a photo sensor, during emission of the at least one priming light pulse with the pulse emitter no sampling of the photo sensor may occur. The priming light pulse may be particularly be adapted to one or more requirements of the optical sensor for making measurements, particularly to bring it into a condition best suited for making accurate measurements with the measurement light pulses. Thus, the priming light pulse may in principle be generated with parameters, which differ from the parameters used for generating measurement light pulses, for example with a different amplitude or duration.

The at least one priming light pulse may be particularly generated with nearly the same parameters as a measurement light pulse, for example nearly the same amplitude, nearly the same duration, nearly the same energy, in order to obtain an accurate stimulation of the signal behaviour of the optical sensor generating measurement light pulses.

In embodiments, the configuring of the optical sensor to generate the at least one priming light pulse may comprise generating the at least one priming light pulse that may have one or more parameter differing from a corresponding parameter for generating the measurement light pulses, wherein particularly the at least one parameter comprises one or more of the following: the energy of priming and measurement light pulses; the duration of priming and measurement light pulses. For example, the priming light pulse may be generated with a lower or higher energy and/or a shorter or longer duration than the measurement light pulses accommodating specific requirements of the optical sensor.

The movable dosage programming component may in embodiments comprise a rotary encoder system having a predefined angular periodicity, and reflections of the measurement light pulses from the rotary encoder system may be detected. The optical sensors' spatial resolutions have a significant impact at transitions of the movable dosage programming component, particularly the rotary encoder system, particularly at transitions of reflecting targets such flags on the movable dosage programming component, particularly the rotary encoder system. The optical sensor does thus usually not detect edges of step changes in the reflectance of targets as abrupt changes in its output signal but as blurred or smoothed transitions. This results in sensor output signals with amplitudes that are position dependent around transitions of the movable dosage programming component, particularly the rotary encoder system such as flag transitions. This may also mean that a mechanical transition point, particularly a flag transition point may not be coincident with that detected by the sensor output signal. In embodiments, the method disclosed herein however may allow to configure the optical sensor to generate the measurement light pulses with different pulse rates. A higher pulse rate may be particularly used for sampling edges or transitions of the rotary encoder system with a higher spatial resolution than a lower pulse rate. The application of different pulse rates may particularly serve to save energy, for example by using a higher pulse rate only for sampling targets of the movable dosage programming component, particularly of edges or transitions of the rotary encoder system.

The term "light pulse" as used herein may comprise electromagnetic radiation pulses within a portion of the electromagnetic spectrum that may comprise ultraviolet light, visible light, and infrared light. Thus, the term "light pulse" may not be construed as being restricted to visible light. In fact, a "light pulse" in the context of this disclosure may mean any electromagnetic radiation pulse, which may be detected with a compatible detector in particular an optical sensor.

In embodiments, the measurement light pulses may be generated either with a first pulse rate or with at least one secondary pulse rate, wherein the first pulse rate is lower than the at least one secondary pulse rate and the at least one priming light pulse is generated with one of the at least one secondary pulse rate and the optical sensor may be configured to generate the at least one priming light pulse at least before generating measurement light pulses with the first pulse rate, wherein each priming light pulse is generated at a time shift prior to a subsequent measurement light pulse corresponding to the at least one secondary pulse rate. A change of the pulse rate may cause a shift in the output signal from the optical sensor particularly due to sensor characteristics, for example when phototransistors are employed in the optical sensor. To eliminate or at least mitigate an occurrence of such a shift, one or more priming light pulses may be generated at least before generating measurement light pulses with the first pulse rate. Thus, the disclosed driving method for optical sensors allows to at least mitigate a variation in sensor output signal amplitudes resulting from different pulse rates used to generate measurement light pulses. This may reduce measurement uncertainty caused by small shifts in the output of an optical sensor, particularly in an output of a phototransistor of an optical sensor, whilst also energy usage of the optical sensor may be minimised by reducing the occurrences of unnecessary changings of the pulse rates due to variations of sensor output signals, which may trigger the switching of pulse rates. It could be found that the generating of the priming light pulse at a time shift prior to the measurement light pulses corresponding to the at least one secondary pulse rate allows to obtain an optimum of compensation of possible undesired effects caused by pulse rate switching.

In further embodiments, the configuring of the optical sensor to generate the measurement light pulses either with the first pulse rate or with the at least one secondary pulse rate may comprise switching the pulse rate depending on exceeding or undercutting at least one predefined threshold by an output signal of the optical sensor. The at least one threshold may be particularly selected to allow a switching of the pulse rate such that a transition may be detected at least partly with a higher spatial resolution. The at least one predefined threshold may be a static threshold, particularly selected by test measurements or determined by some calibration.

In an embodiment, the optical sensor may be configured to generate the measurement light pulses with the first pulse rate if the output signal is below the at least one predefined threshold and to generate the measurement light pulses with the at least one secondary pulse rate if the output signal is above the at least one predefined threshold. For example, the at least one threshold may be selected as lower than the typical reflection of a measurement light pulse from a target of the rotary encoder system, but higher than without a reflection so that at the begin of a transition to a target of the rotary encoder system the pulse rate can be switched from the first to at least one secondary pulse rate, and at the end of the transition it may be switched back to the first pulse rate. Thus, sampling of transitions may be performed with a higher spatial resolution resulting in a more accurate output signal of the optical sensor.

In a further embodiment, a single secondary pulse rate and a single threshold may be provided and the optical sensor may be configured to switch the generation of the measurement light pulses between the first and the secondary pulse rate when the threshold is crossed by the output signal of the optical sensor. This requires less effort for an implementation compared to an implementation with several secondary pulse rates and several thresholds, and it may generate results with an accuracy sufficient for some applications.

In a yet further embodiment, the optical sensor may be configured to generate the measurement light pulses with the first pulse rate when the output signal of the optical sensor is above the threshold and to generate the measurement light pulses with the secondary pulse rate when the output signal of the optical sensor is below the threshold or vice versa.

In still further embodiments, the method may comprise altering the at least one threshold particularly depending on the detected reflections. This allows to adapt the threshold for example to the reflectivity of the rotary encoder system, particularly of targets of the rotary encoder system. When the reflectivity decreases over time, for example due to dust, the at least one threshold may be for example reduced to accommodate the reduced reflectivity. Also, for a possible degradation of the optical sensor the altering of the at least one threshold may be provided.

In yet further embodiments, the optical sensor may be configured to generate the measurement light pulses with the first pulse rate by default. Thus, the method may start with a minimum of energy requirement for measuring, which may preserve for example battery power of an injection device. In alternative embodiments, the optical sensor may be configured to generate the measurement light pulses with the secondary pulse rate by default.

In an embodiment, the first pulse rate may be about 2 milliseconds and the secondary pulse rate and the time shift may be about 250 microseconds. This specific implementation may be particularly suitable for an injection device such as the devices described in WO2014033195.

In embodiments, the method may further comprise at least one of the following: modulating the duration of the measurement light pulses; modifying the amplitude and / or shape of the output signal of the optical sensor; modulating the energy of the measurement light pulses. These measures may further mitigate the uncertainty of measurements caused by pulse rate switching.

In another aspect the present disclosure provides a device for driving an optical sensor of a drug delivery device or a drug delivery add-on device, the device being configured to implement a method as disclosed herein, the device particularly comprising a controller, particularly a microcontroller, the controller being configured by a program to implement the method as disclosed herein. The program may be for example part of a firmware of a controller of an electronics of a drug delivery device or add-on device.

In an embodiment, the device may be configured to determine a dosage delivered and/or selected with the drug delivery device based on detected reflections of the measurement light pulses from the movable dosage programming component.

In a further aspect the present disclosure provides a sensor unit of a drug delivery device or of a drug delivery add-on device, the sensor unit comprising one or more optical sensors driven by a device as disclosed herein, wherein the sensor unit is provided and configured for integration in a drug delivery device or a drug delivery add-on device. The sensor unit may for example comprise a printed circuit board (PCB) with the electronics comprising the controller and further electronic components required for operation of the controller and the at least one optical sensor, and the at least one optical sensor may be wired with the PCB.

In a yet further aspect the present disclosure provides a drug delivery device or a drug delivery add-on device, wherein the drug delivery device comprises a movable dosage programming component and the drug delivery device or the drug delivery add-on device comprises a sensor unit as disclosed herein.

### Brief Description of the Figures

Figure 1 shows an injection device according to an embodiment;
Figure 2 is an elevated side view of a rotary encoder system;
Figure 3 is a plan view of the encoder system shown in Figure 2;
Figure 4 shows a schematic block diagram of an embodiment of a device controller;
Figure 5 shows an example sequence of light pulse generated for driving an optical sensor and an example trace of the output signal of the optical sensor according to an embodiment; and
Figure 6 shows a detailed view of the example sequence from Figure 5.

### Detailed Description of Some Embodiments

In the following, embodiments of the present disclosure will be described with reference to injection devices, particularly an injection device in the form of a pen. The present disclosure is however not limited to such application and may equally well be deployed with other types of drug delivery devices, particularly with another shape than a pen. All absolute values are herein shown by way of example only and should not be construed as limiting.

Examples of injection devices where an injection button and grip are combined are described in WO2014033195. Other examples of injection devices having separate injection button and dial grip components are disclosed e.g in WO2004078239.

In the following discussion, the terms "distal", "distally" and "distal end" refer to the end of an injection pen towards which a needle is provided. The terms "proximal", "proximally" and "proximal end" refer to the opposite end of the injection device towards which an injection button or dosage knob is provided.

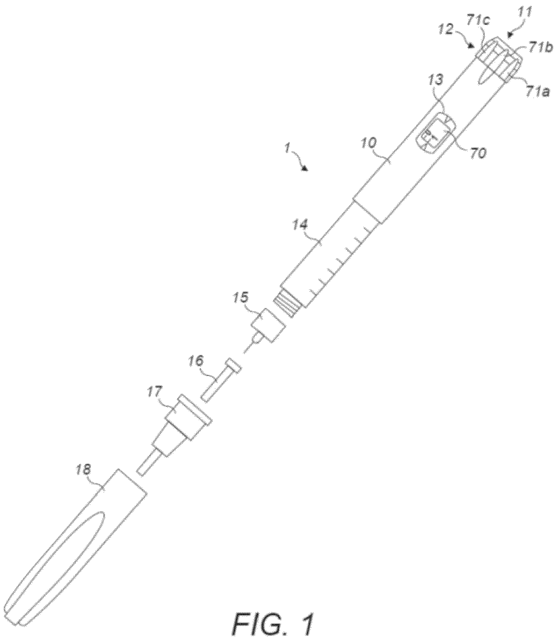

Figure 1 is an exploded view of an injection pen 1, such as the injection pens described in WO2014033195. The injection pen 1 of Figure 1 is a pre-filled, disposable injection pen that comprises a housing 10 and contains an insulin container 14, to which a needle 15 can be affixed. The needle is protected by an inner needle cap 16 and either an outer needle cap 17 other cap 18. An insulin dose to be ejected from injection pen 1 can be programmed, or 'dialled in' by turning a dosage knob 12, and a currently programmed dose is then displayed via dosage window 13, for instance in multiples of units. For example, where the injection pen 1 is configured to administer human insulin, the dosage may be displayed in so-called International Units (IU), wherein one IU is the biological equivalent of about 45.5 micrograms of pure crystalline insulin (1/22 mg). Other units may be employed in injection devices for delivering analogue insulin or other medicaments. It should be noted that the selected dose may equally well be displayed differently than as shown in the dosage window 13 in Figure 1.

The dosage window 13 may be in the form of an aperture in the housing 10, which permits a user to view a limited portion of a dial sleeve 70 that is configured to move when the dosage knob 12 is turned, to provide a visual indication of a currently programmed dose. The dosage knob 12 is rotated on a helical path with respect to the housing 10 when turned during programming. In this example, the dosage knob 12 includes one or more formations 71a, 71b, 71c to facilitate attachment of a data collection device (drug delivery or injection add-on device).

The injection pen 1 may be configured so that turning the dosage knob 12 causes a mechanical click sound to provide acoustical feedback to a user. The dial sleeve 70 mechanically inter-acts with a piston in insulin container 14. In this embodiment, the dosage knob 12 also acts as an injection button. When needle 15 is stuck into a skin portion of a patient, and then dosage knob 12 is pushed in an axial direction, the insulin dose displayed in display window 13 will be ejected from injection pen 1. When the needle 15 of injection pen 1 remains for a certain time in the skin portion after the dosage knob 12 is pushed, a high percentage of the dose is actually injected into the patient's body. Ejection of the insulin dose may also cause a mechanical click sound, which is however different from the sounds produced when rotating the dosage knob 12 during dialling of the dose.

In this embodiment, during delivery of the insulin dose, the dosage knob 12 is returned to its initial position in an axial movement, without rotation, while the dial sleeve 70 is rotated to return to its initial position, e.g. to display a dose of zero units.

Injection pen 1 may be used for several injection processes until either the insulin container 14 is empty or the expiration date of the medicament in the injection pen 1 (e.g. 28 days after the first use) is reached.

Furthermore, before using injection pen 1 for the first time, it may be necessary to perform a so-called "prime shot" to remove air from insulin container 14 and needle 15, for instance by selecting two units of insulin and pressing dosage knob 12 while holding injection pen 1 with the needle 15 upwards. For simplicity of presentation, in the following, it will be assumed that the ejected amounts substantially correspond to the injected doses, so that, for instance the amount of medicament ejected from the injection pen 1 is equal to the dose received by the user. Nevertheless, differences (e.g. losses) between the ejected amounts and the injected doses may need to be taken into account.

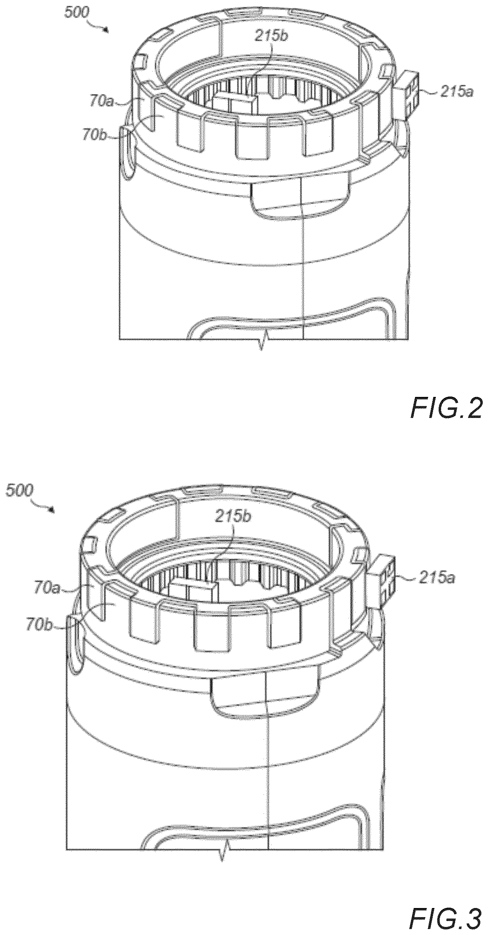

As explained above, the dosage knob 12 also functions as an injection button so that the same component is used for dialling and dispensing. A sensor arrangement 215 (Figures 2 and 3) comprising one or more optical sensors may be mounted in the injection button or dosage knob 12 which is configured to sense the relative rotational position of the dial sleeve 70 relative to the injection button 12. This relative rotation can be equated to the size of the dose dispensed or delivered and used for the purpose of generating and storing or displaying dose history information. The sensor arrangement 215 may comprise a primary (optical) sensor 215a and a secondary (optical) sensor 215b. The sensor arrangement 215 may be also mounted in drug delivery or injection add-on device, which may be provided for usage with different injection devices 1 and configured to collect data acquired with the sensor arrangement 215.

The optical sensors 215a, 215b of the sensor arrangement 215 may be employed with an encoder system, such as the system 500 shown in Figures 2 and 3. The encoder system is configured for use with the device 1 described above and may have a predefined angular periodicity as described in the following. As shown in Figure 2 and Figure 3, the primary sensor 215a and secondary sensor 215b are configured to target specially adapted regions at the proximal end of the dial sleeve 70. In this embodiment, the primary sensor 215a and secondary sensor 215b are IR reflective sensors. Therefore, the specially adapted proximal regions of the dial sleeve 70 are divided into a reflective area 70a and a non-reflective (or absorbent) area 70b resulting in the predefined angular periodicity. The part of the dial sleeve 70 comprising the reflective area 70a and a non-reflective (or absorbent) area 70b may be termed an encoder ring, which has as shown in Figures 2 and 3 the predefined angular periodicity defined by the areas 70a, 70b.

The optical sensor 215a, 215b may comprise a pulse emitter, particularly a LED, and a photo sensor, particularly a photodiode or phototransistor. The pulse emitter and photo sensor may be integrated in a single housing, particularly as a single chip solution, or they may be discrete electronic components. It should be noted that the pulse emitter and the photo sensor may be independently operated, particularly they may be controlled with separate signals and clocks. The term "pulse rate" as used herein may be particularly understood as the pulse rate of the pulse emitter, for example with which pulse clock or frequency the light pulses for measurement and/or priming light pulses are emitted. The photo sensor may be sampled, i.e. controlled with a signal having a sampling rate, which particularly means that the photo sensor is read out with the sampling rate or the output signal of the photo sensor is generated with the sampling rate. The pulse rate and the sampling rate may be identical, or they may differ. The pulse rate and the sampling rate may generate signals that are coincident or the pulse rate and the sampling rate may generate signals that are temporally displaced or the pulse rate and the sampling rate may generate signals that have different durations for example comprise a phase shift and/or the emitter pulse duration may differ from the sample time of the sensor. Particularly during emission of the at least one priming light pulse with the pulse emitter no sampling of the photo sensor may occur since the at least one priming light pulse may particularly not be provided for the purpose of creating a sample of the photo sensor output signal, but to generate a kind of "steady state" of the photo sensor.

To keep production costs to a minimum, it may be favourable to form these areas 70a, 70b from injection moulded polymer. In the case of polymer materials, the absorbency and reflectivity could be controlled with additives, for example carbon black for absorbency and titanium oxide for reflectivity. Alternative implementations are possible whereby the absorbent regions are moulded polymer material and the reflective regions are made from metal (either an additional metal component, or selective metallisation of segments of the polymer dial sleeve 70).

Having two sensors facilitates a power management technique described below. The primary sensor 215a is arranged to target a series of alternating reflective regions 70a and non-reflective regions 70b at a frequency commensurate with the resolution required for the dose history requirements applicable to a particular drug or dosing regime, for example, 1 IU. The secondary sensor 215b is arranged to target a series of alternating reflective regions 70a and non-reflective regions 70b at a reduced frequency compared to the primary sensor 215a. It should be understood that the encoder system 500 could function with only a primary sensor 215a to measure the dispensed dose. The secondary sensor 215b facilitates the power management technique described below.

The two sets of encoded regions 70a, 70b are shown in Figures 2 and 3 concentrically with one external and the other internal. However, any suitable arrangement of the two encoded regions 70a, 70b is possible. Whilst the regions 70a, 70b are shown as castellated regions, it should be borne in mind that other shapes and configurations are possible.

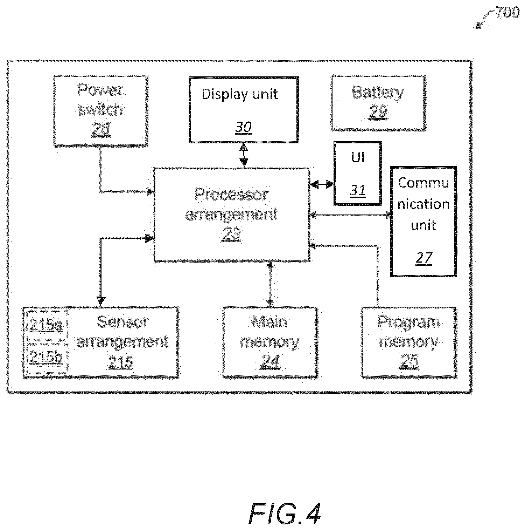

The device 1 or an add-on device for attachment to the device 1 may also include a sensor unit 700, as shown schematically in Figure 4. The sensor unit 700 may comprise the sensor arrangement 215 including the two sensors 215a, 215b and a device for controlling the sensor arrangement 215. The controlling of the sensor arrangement 215 may particularly comprise a driving of at least one of the optical sensors 215a, 215b, wherein driving particularly means how to control an optical sensor to generate light pulses for measurement of a rotation of the encoder ring and to detect reflections of these measurement light pulses from the reflective areas 70a. The controlling device may comprise a processor arrangement 23 including one or more processors, such as a microprocessor, a Digital Signal Processor (DSP), Application Specific Integrated Circuit (ASIC), Field Programmable Gate Array (FPGA) or the like, memory units 24, 25, including program memory 24 and main memory 25, which can store software for execution by the processor arrangement 23, a communication unit or output 27, which may be a wireless communications interface for communicating with another device via a wireless network such as Wi-Fi^{™} or Bluetooth^{®}, and/or an interface for a wired communications link, such as a socket for receiving a Universal Serial Bus (USB), mini-USB or micro-USB connector, a display unit 30, for example a LCD (Liquid Crystal Display), one or more LEDs, and/or an electronic paper display, a user interface (UI) 31, for example one or more buttons and/or touch input devices, a power switch 28, and a battery 29.

The controlling device components 23, 24, 25, 27, 28, 29, 30, 31 may be soldered on a PCB containing the wiring of components. The sensor arrangement 215 may be also attached to the PCB, or may be wired with the processor arrangement 23. The implementation of the sensor unit 700 depends on the drug delivery device or drug delivery add-on device, in which it should be integrated. For example, a PCB with the components 23, 24, 25, 27, 28, 29, 30, 31 may be integrated in the distal end of the injection device 1, and the sensors 215a, 215b may be arranged as shown in Figures 2 and 3 and connected to the PCB via wires. At least some of the components 23, 24, 25, 27 may be also comprised by a SoC (System on Chip) or microcontroller.

A firmware stored in the program memory 25 may configure the processor arrangement 23 to control the sensor arrangement 215 such that expelling of a drug dose being delivered with the device 1 can be detected and the sensors 215a, 215b each output a sensor signal corresponding to the detected delivered drug dose, particularly as described above with regard to the Figures 2 and 3. The processor arrangement 23 receives the sensor signal of each of the sensors 215a, 215b and takes readings of each sensor signal, which are processed to calculate the delivered dose. A reading may comprise for example one or more voltage samples of an analogue voltage signal of the sensor 215a, 215b. A reading may also comprise an integration of an analogue voltage signal of the sensor 215a, 215b over a certain time span. Instead of voltage signals, also electric currents, electric charges or another output signal generated by a sensor may be used for taking readings, for example frequencies of a sensor signal, frequency shifts. The readings may be taken by each sensor 215a, 215b during operation of the injection device 1 to measure the number of units dispensed by the device 1. The measuring of the number of dispensed units may comprise counting peaks of each sensor signal and deriving from the counted peaks the delivered dose as described below by in more detail.

It is advantageous to be able to minimise the power usage of the encoder system 500 so that the size of a battery 29 needed to be packaged into the device 1 can be minimised. The sensors 215a, 215b used in this embodiment require a certain amount of power to operate. This embodiment is arranged such that the sensors 215a, 215b can be switched on and off intermittently at a controlled frequency (i.e. in a strobe-sampling mode). There is inherently a limit to the maximum rotational speed that can be counted by a sampled encoder system before aliasing occurs. Aliasing is the phenomenon where the sampling rate is less than the rate at which sensed regions pass the sensor which means that a miscount could occur when a region change is missed. The secondary sensor 215b with a reduced frequency compared to the primary frequency 215a can tolerate a higher rotational speed before it too becomes aliased. Whilst the secondary sensor 215b is not able to resolve the dose dispensed to the same resolution as the primary sensor 215a, the output of the secondary sensor 215b remains reliable at higher speeds. Therefore both sensors 215a, 215b are used in combination to be able to accurately determine dose delivered up to a first threshold rotational (dispensing) speed. The sensors 215a, 215b can then be used to determine an approximate dose delivered up to a second (higher) threshold dosing speed. At speeds above the second threshold speed the sensors 215a, 215b will not be able to accurately or approximately determine the dose delivered, therefore the second threshold is set above a speed which is not physically possible in the injection pen 1.

The first speed threshold is determined by the pulse rate of primary sensor 215a and the frequency of encoder region transitions, which is fixed at the resolution required by the intended drug or dosing regime (for example one transition per 1 IU). The second speed threshold is determined by the pulse rate of the secondary sensor 215b and the frequency of encoder region transitions. The first threshold is set such that the largest range of dispensing speeds can be covered by the system for accurate reporting of dose dispensed.

The example embodiment shown in Figure 3 has primary sensor 215a targeting region transitions at 1 transition per 1 IU of dose delivered and the secondary sensor 215b targeting region transitions at 1 transition per 6 IU of dose delivered. Other options are possible which include 1 transition per 2 IU, 1 transition per 4 IU, 1 transition per 8 IU and 1 transition per 12 IU units. These options are each possible because there are 24 separate regions 70a, 70b per revolution in the encoder system 500 shown in Figure 3. In general, if the number of separate regions 70a, 70b per revolution were *n* units then there would be options at one region transition per *m* units where *m* was any integer factor of *n* greater than 1 and less than *n.*

The slower the sampling frequency of both sensors 215a, 215b, the lower the power consumption required and therefore the smaller the required size of the battery 29. It is therefore optimal to minimise, by design, the sampling frequency as far as is practical.

The firmware stored in the program memory 25 and being executed by the processor arrangement 23 for detecting the delivered dose is also implemented to configure the optical sensor 215a and/or 215b to generate measurement light pulses, i.e. to drive the optical sensors 215a, 215b. The optical sensor 215a, 215b can be configured by the processor arrangement 23 to generate light pulses with different pulse rates, particularly with a first pulse rate or with at least one secondary pulse rate. The first pulse rate is hereby lower than the at least one secondary pulse rate. The pulse rate determines the time interval of two consecutive light pulses.

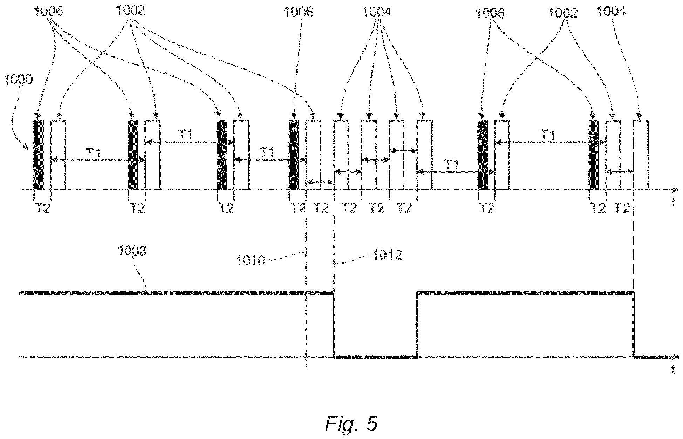

Figure 5 shows an example of a sequence of light pulses 1000 generated by the optical sensor 215a, 215b driven by the processor arrangement 23 configured by the firmware. The shown sequence of light pulses 1000 comprises a first sequence of light pulses 1002 with a first or "slow" pulse rate with a time interval T1 between consecutive pulses 1002. The time interval T1 may be in a specific embodiment an injection pen as described e.g.in WO2014033195 for example about 2 milliseconds, i.e. the time interval between two consecutive light pulses 1002 is about 2 milliseconds. This first sequence of light pulses 1002 may be particularly generated by default and adapted to the encoder ring characteristics, particularly the number of areas 70a, 70b, their dimensions and the distances between reflective areas 70a. It may be selected as a compromise between energy consumption required for sampling and spatial resolution required for obtaining a sensor output signal representing the transitions between the areas 70a and 70b.

The sequence of light pulses 1000 may also comprise a second sequence of light pulses 1004 with second or "fast" pulse rate with a time interval T2 between consecutive pulses 1004. The time interval T2 may be in a specific embodiment of an injection pen as described e.g. in WO2014033195 for example about 250 microseconds, i.e. the time interval between two consecutive light pulses 1004 is 250 microseconds and, thus, much smaller than the time interval T1 between the light pulses 1002. The higher pulse rate of the light pulses 1004 allows a higher spatial resolution than the lower pulse rate of the light pulses 1002, and is therefore particularly be suitable for detecting the transitions between the areas 70a and 70b with a higher accuracy than with the light pulses 1002.

Even if only four "fast" light pulses 1004 are shown in Figure 5, it should be appreciated that more or less "fast" light pulses 1004 may be provided, particularly 16 light pulses, which may be for example applied in an implementation with an injection device as described in WO2014033195.

It should be noted that further sequences of light pulses with different pulse rates, for example higher pulse rates than the first and second pulse rate may be provided in order to obtain even higher spatial resolutions.

The output signal 1008 of the optical sensor is shown in the below example trace in Figure 5. The output signal 1008 represents the detected encoder ring transitions between the areas 70a and 70b. When a transition between areas 70a and 70b is sampled or detected with the optical sensor, the output signal 1008 also changes as shown in Figure 5 at the time point 1012, which represents an ideal edge detection of a transition between areas 70a, 70b.

As outlined above, the driving scheme with the two or more different pulse rates particularly allows to increase the spatial resolution in areas of the encoder ring, where transitions between the areas 70a and 70b have to be detected. Particularly, the firmware may configure the processor arrangement 23 such that the pulse rate is switched from the first to the second pulse rate when a transition between an area 70a and 70b should be sampled with a higher spatial resolution than the resolution achievable with the first pulse rate. The optical sensors' spatial resolutions may have a significant impact at flag transitions or transitions between the areas 70a and 70b. The optical sensor does usually not detect edges of step changes in the target reflectance as it may appear in a transition between the areas 70a and 70b as abrupt changes in its output signal but as blurred or smoothed transition. This may result in sensor output signals with amplitudes that are position dependent around the flag transitions. It may also mean that the mechanical flag transition point may not be coincident with that detected by the optical sensor. Thus, a switching to a higher pulse rate when a transition between the areas 70a and 70b appears in the detection range of the optical sensor 215 may result in a more accurate sensor signal, particularly in a sensor signal, which may more accurately represent a detected transition.

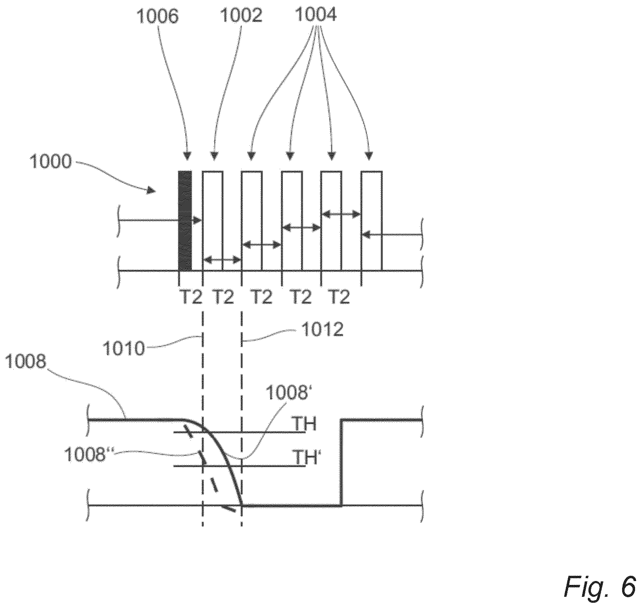

The switching between the pulse rates may be controlled by one or more thresholds as shown in the more detailed view of the transition detection in Figure 6. Two exemplary predefined thresholds TH and TH' are shown in Figure 6. Each predefined threshold TH, TH' may be provided to trigger the switching of the pulse rate from T1 to T2 to obtain a better spatial resolution. Threshold TH is larger than threshold TH'. Thresholds TH, TH' may be for example voltage or current values of an output voltage or current, respectively, of a light receiver of the optical sensor, for example a phototransistor, a photodiode.

The firmware may configure the processor arrangement 23 to process the output signal of the optical sensor by comparing it with at least one of the thresholds TH, TH'. When the processor arrangement 23 detects that the output signal of the optical sensor exceeds or undercuts the threshold TH, TH' (depending on the behaviour of the output signal when a transition between the areas 70a and 70b is detected), the processor arrangement 23 may switch the pulse rate from the first to the second one, i.e. increase the pulse rate by generating light pulses 1004 with a smaller time interval T2, in order to increase the spatial resolution.

The larger threshold TH may be for example provided for steeper transitions between the areas 70a and 70b, for example when the output signal of the optical sensor changes with a higher gradient. This is shown in Figure 6 by the exemplary trace 1008'. The smaller threshold TH' may be for example provided for smoother transitions between the areas 70a and 70b, for example when the output signal of the optical sensor changes with a lower gradient, as shown in Figure 6 by the exemplary trace 1008". The exceeding or undercutting of both thresholds TH, TH' occurs at time point 1010 and incurs a pulse rate switching occurring at time point 1012.

The threshold TH, TH' may be predefined, but it is also possible to adapt them to specific implementations of the encoder ring, for example to better adapt the switching of the pulse rate(s) to the reflectivity of the reflective areas 70a. For example, the threshold TH, TH' may be adapted with a calibration procedure, during which it may be for example adapted to the reflectivity.

Even if in Figure 6 the thresholds TH, TH' are undercut by the output signal 1008 it will be appreciated that also an exceeding of the thresholds TH, TH' by the output signal 1008 may be detected, particularly when the trace of the output signal 1008 is reversed, i.e. it raises from a low to a high value when a transition from a non-reflective area 70b to a reflective area 70a should be detected, which is in Figures 5 and 6 shown by the second transition from the low to the high value of signal 1008.

It is also possible to alter further parameters of the method for driving the optical sensor. The firmware may configure the processor arrangement 23 particularly to modulate the duration of the light pulses 1002 and 1004. For example, the duration may be modulated with the speed of rotation of the encoder ring in order to better adapt the measurement light pulses 1002 and 1004 to higher or lower rotational speeds. A further parameter is the amplitude of the output signal, which can also be modulated particularly depending on the pulse rate. For example, when the pulse rate is switching from a lower pulse rate to a higher one, the amplitude of the output signal may be increased to mitigate a variation due to the pulse rate switching.

Next, a measure to mitigate influences due to pulse rate switching in the output signal of the optical sensor is described. These influences are explained first: when the pulse rate changes there usually occurs a small shift in the optical sensor's output signal, for example in a phototransistor output due to the sensor characteristics. If the encoder ring is positioned such that an optical sensor is close to a flag transition (transition between areas 70a and 70b) such that the output signal is close to the threshold, for example TH or TH' from Figure 6, then it is conceivable that small variations in the amplitude of the output signal due to a change in pulse rate could cause the procedure to remain in the high pulse rate. For example in an implementation with two pulse rates, if the threshold is crossed, samples at a high pulse rate will occur after which it will drop to the low pulse rate, resulting in a small drop in amplitude which may cause the threshold to be tripped, switching it back to the high pulse rate. Such a situation would cause flickering in an implementation of the encoder ring with a Gray code which may add to the uncertainty of the measurement. Energy would be also wasted by unnecessarily sampling at the high rate.

This may be mitigated by the inclusion of a priming light pulse, for example a priming IR LED drive pulse prior to the light pulses used for a measurement. In Figures 5 and 6, the priming light pulse is denoted with reference numeral 1006 and is generated with the time interval T2 before the measurement light pulse 1002, the same period as the fast or second pulse rate of the light pulses 1004. It could be shown that for an optimum compensation this period should match the period of the fast or second pulse rate.

The priming light pulse 1006 can be generated with the same parameters as the measurement light pulses 1002 and/or 1004, particularly with the same duration and energy (particularly amplitude and/or emission spectrum). However, it is also possible and may be advantageous if the priming light pulse 1006 differs in one or more parameters from the measurement light pulses 1002 and/or 1004, for example in order to better mitigate the undesired influence(s) caused by the pulse rate switching. For example, the duration and/or energy of the priming light pulse may differ from that of the measurement light pulses in order to adapt the priming light pulse to the optical sensor characteristics. Particularly, differing duration and/or energy of the priming light pulse may better suited to mitigate shifts in the optical sensor's output signal depending on the sensor's characteristics, particularly a shorter duration and a lower energy than the measurement light pulses may sufficiently mitigate the optical sensor's output signal shift when the pulse rate switches.

A mitigation could also be implemented by appropriately modulating the drive pulse durations for generating the light pulses, or altering the threshold(s), or modifying the output signal(s) amplitude(s) of the optical sensor(s).

The herein disclosed driving method for optical sensors is particularly suitable for injection devices comprising a movable dosage programming component with a rotary encoder system having a predefined angular periodicity such as the injection pens as described in WO2014033195. However, it will be appreciated that the disclosed driving method is generally applicable to drug delivery devices having a movable dosage programming component with a rotary encoder system having a predefined angular periodicity. The disclosed method can also be applied to a drug delivery add-on device, for example a wireless communication device provided for attachment to a drug delivery device such as an injection device and designed and configured to provide electronic functions such as dose recording and/or connectivity with external electronic devices. Such an add-on device may for example comprise electronics with the sensor unit 700 as shown in Figure 4 and be designed to be able to measure dosage selected and/or delivered with the drug delivery device to which the add-on device is attached. The electronics comprised by the add-on device may then implement the method disclosed herein, particularly by means of a firmware configuring a controller such as a microcontroller for driving one or more optical sensor(s) provided for generating the measurement light pulses for detecting dosage selection and/or expelling. Implementations of optical encoder systems, for which the herein disclosed method is particularly suited, are disclosed for example in the above mentioned WO2019101962A1.

The herein disclosed method allows to generate an output signal of an optical sensor, which accurately represents the movement of a movable dosage programming component. This may be particularly obtained by switching a pulse rate, with which measurement light pulses are generated to sample transitions between different areas of a rotary encoder system, from a first lower pulse rate to at least one secondary higher pulse rate. The switching may be particularly triggered by crossing of one or more thresholds by the output signal of an optical sensor. In order to mitigate influences on the output signal caused by the pulse rate switching, herein disclosed embodiments suggest to introduce a priming light pulse generated before the measurement light pulses with the first pulse rate. The priming light pulse is particularly generated at a time shift prior to the measurement light pulse, which corresponds to the at least one secondary pulse rate.

The terms "drug" or "medicament" are used synonymously herein and describe a pharmaceutical formulation containing one or more active pharmaceutical ingredients or pharmaceutically acceptable salts or solvates thereof, and optionally a pharmaceutically acceptable carrier. An active pharmaceutical ingredient ("API"), in the broadest terms, is a chemical structure that has a biological effect on humans or animals. In pharmacology, a drug or medicament is used in the treatment, cure, prevention, or diagnosis of disease or used to otherwise enhance physical or mental well-being. A drug or medicament may be used for a limited duration, or on a regular basis for chronic disorders.

As described below, a drug or medicament can include at least one API, or combinations thereof, in various types of formulations, for the treatment of one or more diseases. Examples of API may include small molecules having a molecular weight of 500 Da or less; polypeptides, peptides and proteins (e.g., hormones, growth factors, antibodies, antibody fragments, and enzymes); carbohydrates and polysaccharides; and nucleic acids, double or single stranded DNA (including naked and cDNA), RNA, antisense nucleic acids such as antisense DNA and RNA, small interfering RNA (siRNA), ribozymes, genes, and oligonucleotides. Nucleic acids may be incorporated into molecular delivery systems such as vectors, plasmids, or liposomes. Mixtures of one or more drugs are also contemplated.

The drug or medicament may be contained in a primary package or "drug container" adapted for use with a drug delivery device. The drug container may be, e.g., a cartridge, syringe, reservoir, or other solid or flexible vessel configured to provide a suitable chamber for storage (e.g., short-or long-term storage) of one or more drugs. For example, in some instances, the chamber may be designed to store a drug for at least one day (e.g., 1 to at least 30 days). In some instances, the chamber may be designed to store a drug for about 1 month to about 2 years. Storage may occur at room temperature (e.g., about 20°C), or refrigerated temperatures (e.g., from about - 4°C to about 4°C). In some instances, the drug container may be or may include a dual-chamber cartridge configured to store two or more components of the pharmaceutical formulation to-be-administered (e.g., an API and a diluent, or two different drugs) separately, one in each chamber. In such instances, the two chambers of the dual-chamber cartridge may be configured to allow mixing between the two or more components prior to and/or during dispensing into the human or animal body. For example, the two chambers may be configured such that they are in fluid communication with each other (e.g., by way of a conduit between the two chambers) and allow mixing of the two components when desired by a user prior to dispensing. Alternatively or in addition, the two chambers may be configured to allow mixing as the components are being dispensed into the human or animal body.

The drugs or medicaments contained in the drug delivery devices as described herein can be used for the treatment and/or prophylaxis of many different types of medical disorders. Examples of disorders include, e.g., diabetes mellitus or complications associated with diabetes mellitus such as diabetic retinopathy, thromboembolism disorders such as deep vein or pulmonary thromboembolism. Further examples of disorders are acute coronary syndrome (ACS), angina, myocardial infarction, cancer, macular degeneration, inflammation, hay fever, atherosclerosis and/or rheumatoid arthritis. Examples of APIs and drugs are those as described in handbooks such as Rote Liste 2014, for example, without limitation, main groups 12 (antidiabetic drugs) or 86 (oncology drugs), and Merck Index, 15th edition.

Examples of APIs for the treatment and/or prophylaxis of type 1 or type 2 diabetes mellitus or complications associated with type 1 or type 2 diabetes mellitus include an insulin, e.g., human insulin, or a human insulin analogue or derivative, a glucagon-like peptide (GLP-1), GLP-1 analogues or GLP-1 receptor agonists, or an analogue or derivative thereof, a dipeptidyl peptidase-4 (DPP4) inhibitor, or a pharmaceutically acceptable salt or solvate thereof, or any mixture thereof. As used herein, the terms "analogue" and "derivative" refers to a polypeptide which has a molecular structure which formally can be derived from the structure of a naturally occurring peptide, for example that of human insulin, by deleting and/or exchanging at least one amino acid residue occurring in the naturally occurring peptide and/or by adding at least one amino acid residue. The added and/or exchanged amino acid residue can either be codable amino acid residues or other naturally occurring residues or purely synthetic amino acid residues. Insulin analogues are also referred to as "insulin receptor ligands". In particular, the term "derivative" refers to a polypeptide which has a molecular structure which formally can be derived from the structure of a naturally occurring peptide, for example that of human insulin, in which one or more organic substituent (e.g. a fatty acid) is bound to one or more of the amino acids. Optionally, one or more amino acids occurring in the naturally occurring peptide may have been deleted and/or replaced by other amino acids, including non-codeable amino acids, or amino acids, including non-codeable, have been added to the naturally occurring peptide.

Examples of insulin analogues are Gly(A21), Arg(B31), Arg(B32) human insulin (insulin glargine); Lys(B3), Glu(B29) human insulin (insulin glulisine); Lys(B28), Pro(B29) human insulin (insulin lispro); Asp(B28) human insulin (insulin aspart); human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Examples of insulin derivatives are, for example, B29-N-myristoyl-des(B30) human insulin, Lys(B29) (N- tetradecanoyl)-des(B30) human insulin (insulin detemir, Levemir^{®}); B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-gamma-glutamyl)-des(B30) human insulin, B29-N-omega-carboxypentadecanoyl-gamma-L-glutamyl-des(B30) human insulin (insulin degludec, Tresiba^{®}); B29-N-(N-lithocholyl-gamma-glutamyl)-des(B30) human insulin; B29-N-(ω-carboxyheptadecanoyl)-des(B30) human insulin and B29-N-(ω-carboxyheptadecanoyl) human insulin.

Examples of GLP-1, GLP-1 analogues and GLP-1 receptor agonists are, for example, Lixisenatide (Lyxumia^{®}), Exenatide (Exendin-4, Byetta^{®}, Bydureon^{®}, a 39 amino acid peptide which is produced by the salivary glands of the Gila monster), Liraglutide (Victoza^{®}), Semaglutide, Taspoglutide, Albiglutide (Syncria^{®}), Dulaglutide (Trulicity^{®}), rExendin-4, CJC-1134-PC, PB-1023, TTP-054, Langlenatide / HM-11260C (Efpeglenatide), HM-15211, CM-3, GLP-1 Eligen, ORMD-0901, NN-9423, NN-9709, NN-9924, NN-9926, NN-9927, Nodexen, Viador-GLP-1, CVX-096, ZYOG-1, ZYD-1, GSK-2374697, DA-3091, MAR-701, MAR709, ZP-2929, ZP-3022, ZP-DI-70, TT-401 (Pegapamodtide), BHM-034. MOD-6030, CAM-2036, DA-15864, ARI-2651, ARI-2255, Tirzepatide (LY3298176), Bamadutide (SAR425899), Exenatide-XTEN and Glucagon-Xten.

An example of an oligonucleotide is, for example: mipomersen sodium (Kynamro^{®}), a cholesterol-reducing antisense therapeutic for the treatment of familial hypercholesterolemia or RG012 for the treatment of Alport syndrom.

Examples of DPP4 inhibitors are Linagliptin, Vildagliptin, Sitagliptin, Denagliptin, Saxagliptin, Berberine.

Examples of hormones include hypophysis hormones or hypothalamus hormones or regulatory active peptides and their antagonists, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, and Goserelin.

Examples of polysaccharides include a glucosaminoglycane, a hyaluronic acid, a heparin, a low molecular weight heparin or an ultra-low molecular weight heparin or a derivative thereof, or a sulphated polysaccharide, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium. An example of a hyaluronic acid derivative is Hylan G-F 20 (Synvisc^{®}), a sodium hyaluronate.

The term "antibody", as used herein, refers to an immunoglobulin molecule or an antigen-binding portion thereof. Examples of antigen-binding portions of immunoglobulin molecules include F(ab) and F(ab')2 fragments, which retain the ability to bind antigen. The antibody can be polyclonal, monoclonal, recombinant, chimeric, de-immunized or humanized, fully human, non-human, (e.g., murine), or single chain antibody. In some embodiments, the antibody has effector function and can fix complement. In some embodiments, the antibody has reduced or no ability to bind an Fc receptor. For example, the antibody can be an isotype or subtype, an antibody fragment or mutant, which does not support binding to an Fc receptor, e.g., it has a mutagenized or deleted Fc receptor binding region. The term antibody also includes an antigen-binding molecule based on tetravalent bispecific tandem immunoglobulins (TBTI) and/or a dual variable region antibody-like binding protein having cross-over binding region orientation (CODV).

The terms "fragment" or "antibody fragment" refer to a polypeptide derived from an antibody polypeptide molecule (e.g., an antibody heavy and/or light chain polypeptide) that does not comprise a full-length antibody polypeptide, but that still comprises at least a portion of a full-length antibody polypeptide that is capable of binding to an antigen. Antibody fragments can comprise a cleaved portion of a full length antibody polypeptide, although the term is not limited to such cleaved fragments. Antibody fragments that are useful in the present invention include, for example, Fab fragments, F(ab')2 fragments, scFv (single-chain Fv) fragments, linear antibodies, monospecific or multispecific antibody fragments such as bispecific, trispecific, tetraspecific and multispecific antibodies (e.g., diabodies, triabodies, tetrabodies), monovalent or multivalent antibody fragments such as bivalent, trivalent, tetravalent and multivalent antibodies, minibodies, chelating recombinant antibodies, tribodies or bibodies, intrabodies, nanobodies, small modular immunopharmaceuticals (SMIP), binding-domain immunoglobulin fusion proteins, camelized antibodies, and VHH containing antibodies. Additional examples of antigen-binding antibody fragments are known in the art.

The terms "Complementarity-determining region" or "CDR" refer to short polypeptide sequences within the variable region of both heavy and light chain polypeptides that are primarily responsible for mediating specific antigen recognition. The term "framework region" refers to amino acid sequences within the variable region of both heavy and light chain polypeptides that are not CDR sequences, and are primarily responsible for maintaining correct positioning of the CDR sequences to permit antigen binding. Although the framework regions themselves typically do not directly participate in antigen binding, as is known in the art, certain residues within the framework regions of certain antibodies can directly participate in antigen binding or can affect the ability of one or more amino acids in CDRs to interact with antigen.

Examples of antibodies are anti PCSK-9 mAb (e.g., Alirocumab), anti IL-6 mAb (e.g., Sarilumab), and anti IL-4 mAb (e.g., Dupilumab).

Pharmaceutically acceptable salts of any API described herein are also contemplated for use in a drug or medicament in a drug delivery device. Pharmaceutically acceptable salts are for example acid addition salts and basic salts.

Those of skill in the art will understand that modifications (additions and/or removals) of various components of the APIs, formulations, apparatuses, methods, systems and embodiments described herein may be made without departing from the full scope of the present invention, which encompass such modifications and any and all equivalents thereof.

An example drug delivery device may involve a needle-based injection system as described in Table 1 of section 5.2 of ISO 11608-1:2014(E). As described in ISO 11608-1:2014(E), needle-based injection systems may be broadly distinguished into multi-dose container systems and single-dose (with partial or full evacuation) container systems. The container may be a replaceable container or an integrated non-replaceable container.

As further described in ISO 11608-1:2014(E), a multi-dose container system may involve a needle-based injection device with a replaceable container. In such a system, each container holds multiple doses, the size of which may be fixed or variable (pre-set by the user). Another multi-dose container system may involve a needle-based injection device with an integrated non-replaceable container. In such a system, each container holds multiple doses, the size of which may be fixed or variable (pre-set by the user).

As further described in ISO 11608-1:2014(E), a single-dose container system may involve a needle-based injection device with a replaceable container. In one example for such a system, each container holds a single dose, whereby the entire deliverable volume is expelled (full evacuation). In a further example, each container holds a single dose, whereby a portion of the deliverable volume is expelled (partial evacuation). As also described in ISO 11608-1:2014(E), a single-dose container system may involve a needle-based injection device with an integrated non-replaceable container. In one example for such a system, each container holds a single dose, whereby the entire deliverable volume is expelled (full evacuation). In a further example, each container holds a single dose, whereby a portion of the deliverable volume is expelled (partial evacuation).

## Claims

1. A method for driving an optical sensor (215a, 215b) of a drug delivery device (1) or of a drug delivery add-on device, wherein the drug delivery device (1) comprises a movable dosage programming component and the optical sensor (215a, 215b) is provided and configured to detect movement of the movable dosage programming component relative to the sensor arrangement (215) during dosing of a drug by generating measurement light pulses (1000) and detecting reflections of the measurement light pulses from the movable dosage programming component, and wherein the method comprises configuring the optical sensor (215a, 215b) to generate the measurement light pulses (1000) with at least one predefined pulse rate and to generate at least one priming light pulse (1006) before one or more measurement light pulses, wherein the at least one priming light pulse (1006) is generated with one of the at least one predefined pulse rate, and wherein the at least one priming light pulse (1006) differs from a measurement light pulse in that it is not used for making measurements.

2. The method of claim 1, wherein the configuring of the optical sensor to generate the at least one priming light pulse comprises generating the at least one priming light pulse that has one or more parameter differing from a corresponding parameter for generating the measurement light pulses, wherein the at least one parameter comprises one or more of the following: the energy of priming and measurement light pulses; the duration of priming and measurement light pulses.

3. The method of claim 1 or 2, wherein the measurement light pulses are generated either with a first pulse rate or with at least one secondary pulse rate, wherein the first pulse rate is lower than the at least one secondary pulse rate and the at least one priming light pulse is generated with one of the at least one secondary pulse rate and the optical sensor is configured to generate the at least one priming light pulse at least before generating measurement light pulses with the first pulse rate, wherein each priming light pulse is generated at a time shift prior to a subsequent measurement light pulse corresponding to the at least one secondary pulse rate.

4. The method of claim 3, wherein the configuring of the optical sensor to generate the measurement light pulses either with the first pulse rate or with the at least one secondary pulse rate comprises switching the pulse rate depending on exceeding or undercutting at least one predefined threshold by an output signal of the optical sensor.

5. The method of claim 4, wherein the optical sensor is configured to generate the measurement light pulses with the first pulse rate if the output signal is below the at least one predefined threshold and to generate the measurement light pulses with the at least one secondary pulse rate if the output signal is above the at least one predefined threshold.

6. The method of claim 5, wherein a single secondary pulse rate and a single threshold are provided and the optical sensor is configured to switch the generation of the measurement light pulses between the first and the second pulse rate when the threshold is crossed by the output signal of the optical sensor.

7. The method of claim 6, wherein the optical sensor is configured to generate the measurement light pulses with the first pulse rate when the output signal of the optical sensor is above the threshold and to generate the measurement light pulses with the second pulse rate when the output signal of the optical sensor is below the threshold or vice versa.

8. The method of any of claims 4 to 7, further comprising altering the at least one threshold particularly depending on the detected reflections.

9. The method of any of claims 4 to 8, wherein the optical sensor is configured to generate the measurement light pulses with the first or the second pulse rate by default.

10. The method of any of claims 4 to 9, wherein the first pulse rate is about 2 milliseconds and the second pulse rate and the time shift are about 250 microseconds.

11. The method of any preceding claim, further comprising at least one of the following: modulating the duration of the measurement light pulses; modifying the amplitude and / or shape of the output signal of the optical sensor; modulating the energy of the measurement light pulses.

12. A device for driving an optical sensor (215a,215b) of a drug delivery device (1) or a drug delivery add-on device, the device being configured to implement a method of any preceding claim, the device particularly comprising a controller, particularly a microcontroller, the controller being configured by a program to implement the method of any preceding claim.

13. The device of claim 12, being configured to determine a dosage delivered and/or selected with the drug delivery device based on detected reflections of the measurement light pulses from the movable dosage programming component.

14. A sensor unit (700) of a drug delivery device (1) or of a drug delivery add-on device, the sensor unit comprising one or more optical sensors (215a,215b) driven by a device of claim 12 or 13, wherein the sensor unit is provided and configured for integration in a drug delivery device or a drug delivery add-on device.

15. A drug delivery device (1) or a drug delivery add-on device, wherein the drug delivery device comprises a movable dosage programming component and the drug delivery device or the drug delivery add-on device comprises a sensor unit (700) of claim 14.

## Patentansprüche

1. Verfahren zum Ansteuern eines optischen Sensors (215a, 215b) einer Medikamenten-Verabreichungsvorrichtung (1) oder einer zusätzlichen Medikamenten-Verabreichungsvorrichtung, wobei die Medikamenten-Verabreichungsvorrichtung (1) eine bewegbare Dosierungsprogrammierkomponente umfasst und der optische Sensor (215a, 215b) bereitgestellt und dazu ausgelegt ist, um eine Bewegung der bewegbaren Dosierungsprogrammierkomponente relativ zu der Sensoranordnung (215) während der Dosierung eines Medikaments durch Erzeugen von Messlichtimpulsen (1000) und Detektieren von Reflexionen der Messlichtimpulse von der bewegbaren Dosierungsprogrammierkomponente zu detektieren, und wobei das Verfahren Konfigurieren des optischen Sensors (215a, 215b) umfasst, um die Messlichtimpulse (1000) mit mindestens einer vordefinierten Impulsrate zu erzeugen und mindestens einen Initialisierungslichtimpuls (1006) vor einem oder mehreren Messlichtimpulsen zu erzeugen, wobei der mindestens eine Initialisierungslichtimpuls (1006) mit einer der einen oder mehreren vordefinierten Impulsraten erzeugt wird, und wobei sich der mindestens eine Initialisierungslichtimpuls (1006) von einem Messlichtimpuls dadurch unterscheidet, dass er nicht zum Durchführen von Messungen verwendet wird.

2. Verfahren nach Anspruch 1, wobei das Konfigurieren des optischen Sensors zum Erzeugen des mindestens einen Initialisierungslichtimpulses Erzeugen des mindestens einen Initialisierungslichtimpulses umfasst, der einen oder mehrere Parameter aufweist, die sich von einem entsprechenden Parameter zum Erzeugen der Messlichtimpulse unterscheiden, wobei der mindestens eine Parameter eines oder mehrere der Folgenden umfasst: die Energie von Initialisierungs- und Messlichtimpulsen; die Dauer von Initialisierungs- und Messlichtimpulsen.

3. Verfahren nach Anspruch 1 oder 2, wobei die Messlichtimpulse entweder mit einer ersten Impulsrate oder mit mindestens einer sekundären Impulsrate erzeugt werden, wobei die erste Impulsrate niedriger als die mindestens eine sekundäre Impulsrate ist und der mindestens eine Initialisierungslichtimpuls mit einer der einen oder mehreren sekundären Impulsraten erzeugt wird und der optische Sensor dazu ausgelegt ist, den mindestens einen Initialisierungslichtimpuls mindestens vor dem Erzeugen von Messlichtimpulsen mit der ersten Impulsrate zu erzeugen, wobei jeder Initialisierungslichtimpuls mit einer Zeitverschiebung vor einem nachfolgenden Messlichtimpuls, der der mindestens einen sekundären Impulsrate entspricht, erzeugt wird.

4. Verfahren nach Anspruch 3, wobei das Konfigurieren des optischen Sensors zum Erzeugen der Messlichtimpulse entweder mit der ersten Impulsrate oder mit der mindestens einen sekundären Impulsrate das Schalten der Impulsrate in Abhängigkeit von dem Überschreiten oder Unterschreiten mindestens einer vordefinierten Schwelle durch ein Ausgangssignal des optischen Sensors umfasst.

5. Verfahren nach Anspruch 4, wobei der optische Sensor dazu ausgelegt ist, die Messlichtimpulse mit der ersten Impulsrate zu erzeugen, wenn das Ausgangssignal unter der mindestens einen vordefinierten Schwelle liegt, und die Messlichtimpulse mit der mindestens einen sekundären Impulsrate zu erzeugen, wenn das Ausgangssignal über der mindestens einen vordefinierten Schwelle liegt.

6. Verfahren nach Anspruch 5, wobei eine einzelne sekundäre Impulsrate und eine einzelne Schwelle bereitgestellt werden und der optische Sensor dazu ausgelegt ist, die Erzeugung der Messlichtimpulse zwischen der ersten und der zweiten Impulsrate umzuschalten, wenn die Schwelle durch das Ausgangssignal des optischen Sensors überschritten wird.

7. Verfahren nach Anspruch 6, wobei der optische Sensor dazu ausgelegt ist, die Messlichtimpulse mit der ersten Impulsrate zu erzeugen, wenn das Ausgangssignal des optischen Sensors über der Schwelle liegt, und die Messlichtimpulse mit der zweiten Impulsrate zu erzeugen, wenn das Ausgangssignal des optischen Sensors unter der Schwelle liegt oder umgekehrt.

8. Verfahren nach einem der Ansprüche 4 bis 7, ferner umfassend Ändern der mindestens einen Schwelle, insbesondere in Abhängigkeit von den detektierten Reflexionen.

9. Verfahren nach einem der Ansprüche 4 bis 8, wobei der optische Sensor dazu ausgelegt ist, die Messlichtimpulse standardmäßig mit der ersten oder der zweiten Impulsrate zu erzeugen.

10. Verfahren nach einem der Ansprüche 4 bis 9, wobei die erste Impulsrate etwa 2 Millisekunden beträgt und die zweite Impulsrate und die Zeitverschiebung etwa 250 Mikrosekunden betragen.

11. Verfahren nach einem der vorhergehenden Ansprüche, ferner umfassend mindestens eines der Folgenden: Modulieren der Dauer der Messlichtimpulse; Modifizieren der Amplitude und/oder Form des Ausgangssignals des optischen Sensors; Modulieren der Energie der Messlichtimpulse.

12. Vorrichtung zum Ansteuern eines optischen Sensors (215a,215b) einer Medikamenten-Verabreichungsvorrichtung (1) oder einer zusätzlichen Medikamenten-Verabreichungsvorrichtung, wobei die Vorrichtung dazu ausgelegt ist, ein Verfahren nach einem der vorhergehenden Ansprüche zu implementieren, wobei die Vorrichtung insbesondere eine Steuerung, insbesondere einen Mikrocontroller, umfasst, wobei die Steuerung durch ein Programm zum Implementieren des Verfahrens nach einem der vorhergehenden Ansprüche ausgelegt ist.

13. Vorrichtung nach Anspruch 12, die dazu ausgelegt ist, eine mit der Medikamenten-Verabreichungsvorrichtung abgegebene und/oder ausgewählte Dosis basierend auf detektierten Reflexionen der Messlichtimpulse von der bewegbaren Dosierungsprogrammierkomponente zu bestimmen.

14. Sensoreinheit (700) einer Medikamenten-Verabreichungsvorrichtung (1) oder einer zusätzlichen Medikamenten-Verabreichungsvorrichtung, wobei die Sensoreinheit einen oder mehrere optische Sensoren (215a, 215b) umfasst, die durch eine Vorrichtung nach Anspruch 12 oder 13 angesteuert werden, wobei die Sensoreinheit zur Integration in eine Medikamenten-Verabreichungsvorrichtung oder eine zusätzliche Medikamenten-Verabreichungsvorrichtung bereitgestellt und ausgelegt ist.

15. Medikamenten-Verabreichungsvorrichtung (1) oder zusätzliche Medikamenten-Verabreichungsvorrichtung, wobei die Medikamenten-Verabreichungsvorrichtung eine bewegbare Dosierungsprogrammierkomponente umfasst und die Medikamenten-Verabreichungsvorrichtung oder die zusätzliche Medikamenten-Verabreichungsvorrichtung eine Sensoreinheit (700) nach Anspruch 14 umfasst.

## Revendications

1. Procédé pour commander un capteur optique (215a, 215b) d'un dispositif d'administration de médicament (1) ou d'un dispositif complémentaire d'administration de médicament, dans lequel le dispositif d'administration de médicament (1) comprend un composant de programmation de dosage mobile et le capteur optique (215a, 215b) est prévu et configuré pour détecter un mouvement du composant de programmation de dosage mobile relativement à l'agencement de capteur (215) durant le dosage d'un médicament en générant des impulsions de lumière de mesure (1000) et détectant des réflexions des impulsions de lumière de mesure provenant du composant de programmation de dosage mobile, et dans lequel le procédé comprend la configuration du capteur optique (215a, 215b) pour générer les impulsions de lumière de mesure (1000) avec au moins une fréquence d'impulsions prédéfinie et pour générer au moins une impulsion de lumière d'amorce (1006) avant une ou plusieurs impulsions de lumière de mesure, dans lequel l'au moins une impulsion de lumière d'amorce (1006) est générée avec une de l'au moins une fréquence d'impulsions prédéfinie, et dans lequel l'au moins une impulsion de lumière d'amorce (1006) diffère d'une impulsion de lumière de mesure en ce qu'elle n'est pas utilisée pour réaliser des mesures.

2. Procédé selon la revendication 1, dans lequel la configuration du capteur optique pour générer l'au moins une impulsion de lumière d'amorce comprend la génération de l'au moins une impulsion de lumière d'amorce qui a un ou plusieurs paramètres différant d'un paramètre correspondant pour générer les impulsions de lumière de mesure, dans lequel l'au moins un paramètre comprend un ou plusieurs de ce qui suit : l'énergie d'impulsions de lumière d'amorçage et de mesure ; la durée d'impulsions de lumière d'amorçage et de mesure.

3. Procédé selon la revendication 1 ou 2, dans lequel les impulsions de lumière de mesure sont générées soit avec une première fréquence d'impulsion soit avec au moins une fréquence d'impulsion secondaire, dans lequel la première fréquence d'impulsion est plus basse que l'au moins une fréquence d'impulsion secondaire et l'au moins une impulsion de lumière d'amorce est générée avec une de l'au moins une fréquence d'impulsion secondaire et le capteur optique est configuré pour générer l'au moins une impulsion de lumière d'amorce au moins avant la génération d'impulsions de lumière de mesure avec la première fréquence d'impulsion, dans lequel chaque impulsion de lumière d'amorçage est générée à un décalage temporel avant une impulsion de lumière de mesure subséquente correspondant à l'au moins une fréquence d'impulsion secondaire.

4. Procédé selon la revendication 3, dans lequel la configuration du capteur optique pour générer les impulsions de lumière de mesure soit avec la première fréquence d'impulsion soit avec l'au moins une fréquence d'impulsion secondaire comprend la commutation de la fréquence d'impulsion en fonction du dépassement ou du sous-passement d'au moins un seuil prédéfini par un signal de sortie du capteur optique.

5. Procédé selon la revendication 4, dans lequel le capteur optique est configuré pour générer les impulsions de lumière de mesure avec la première fréquence d'impulsion si le signal de sortie est en dessous de l'au moins un seuil prédéfini et pour générer les impulsions de lumière de mesure avec l'au moins une fréquence d'impulsion secondaire si le signal de sortie est au-dessus de l'au moins un seuil prédéfini.

6. Procédé selon la revendication 5, dans lequel une seule fréquence d'impulsion secondaire et un seul seuil sont prévus et le capteur optique est configuré pour commuter la génération des impulsions de lumière de mesure entre la première et la seconde fréquence d'impulsion lorsque le seuil est croisé par le signal de sortie du capteur optique.

7. Procédé selon la revendication 6, dans lequel le capteur optique est configuré pour générer les impulsions de lumière de mesure avec la première fréquence d'impulsion lorsque le signal de sortie du capteur optique est au-dessus du seuil et pour générer les impulsions de lumière de mesure avec la seconde fréquence d'impulsion lorsque le signal de sortie du capteur optique est en dessous du seuil ou vice versa.

8. Procédé selon l'une quelconque des revendications 4 à 7, comprenant en outre la modification de l'au moins un seuil particulièrement en fonction des réflexions détectées.

9. Procédé selon l'une quelconque des revendications 4 à 8, dans lequel le capteur optique est configuré pour générer les impulsions de lumière de mesure avec la première ou la seconde fréquence d'impulsion par défaut.

10. Procédé selon l'une quelconque des revendications 4 à 9, dans lequel la première fréquence d'impulsion est d'environ 2 millisecondes et la seconde fréquence d'impulsion et le décalage temporel sont de l'ordre de 250 microsecondes.

11. Procédé selon l'une quelconque des revendications précédentes, comprenant en outre au moins une de ce qui suit : la modulation de la durée des impulsions de lumière de mesure ; la modification de l'amplitude et/ou de la forme du signal de sortie du capteur optique ; la modulation de l'énergie des impulsions de lumière de mesure.

12. Dispositif pour commander un capteur optique (215a, 215b) d'un dispositif d'administration de médicament (1) ou d'un dispositif complémentaire d'administration de médicament, le dispositif étant configuré pour mettre en œuvre un procédé selon l'une quelconque des revendications précédentes, le dispositif comprenant particulièrement un contrôleur, particulièrement un microcontrôleur, le contrôleur étant configuré par un programme pour mettre en œuvre le procédé selon l'une quelconque des revendications précédentes.

13. Dispositif selon la revendication 12, configuré pour déterminer un dosage administré et/ou sélectionné avec le dispositif d'administration de médicament sur la base de réflexions détectées des impulsions de lumière de mesure provenant du composant de programmation de dosage mobile.

14. Unité capteur (700) d'un dispositif d'administration de médicament (1) ou d'un dispositif complémentaire d'administration de médicament, l'unité capteur comprenant un ou plusieurs capteurs optiques (215a, 215b) commandés par un dispositif selon la revendication 12 ou 13, dans laquelle l'unité capteur est prévue et configurée pour l'intégration dans un dispositif d'administration de médicament ou un dispositif complémentaire d'administration de médicament.

15. Dispositif d'administration de médicament (1) ou dispositif complémentaire d'administration de médicament, dans lequel le dispositif d'administration de médicament comprend un composant de programmation de dosage mobile et le dispositif d'administration de médicament ou le dispositif complémentaire d'administration de médicament comprend une unité capteur (700) selon la revendication 14.
